# EUROPÄISCHE PATENTSCHRIFT

(11) **EP 1 430 906 B1**
(45) Veröffentlichungstag und Bekanntmachung des Hinweises auf die Patenterteilung: **18.06.2008**
(21) Anmeldenummer: 03028015.0
(22) Anmeldetag: 06.12.2003
(51) Int. Cl.: A61K 8/73, A61K 8/81, A61Q 19/08

(54) **Nanoskalige Hydrogele gegen Falten, raue und trockene Haut**
Nanoscale hydrogels against wrinkles, rough and dry skin
Hydrogels nanométriques contre les rides, peaux rugueuses et sèches

(30) Priorität: 16.12.2002 DE 10258960
(43) Veröffentlichungstag der Anmeldung: 23.06.2004
(73) Patentinhaber: Henkel AG & Co. KGaA, 40589 Düsseldorf (DE)
(72) Erfinder: Banowski, Bernhard, 40597 Düsseldorf (DE); Jekel, Maren, Dr., 40476 Düsseldorf (DE)

(56) Entgegenhaltungen:
- WO-A-00/07603
- WO-A-95/24430
- WO-A-02/076392
- US-A- 6 159 457
- US-A1- 2002 187 173

## Beschreibung

Gegenstand der vorliegenden Erfindung ist die Verwendung nanoskaliger Hydrogele in kosmetischen Hautpflegezusammensetzungen zur nicht-therapeutischen Behandlung von Falten und Fältchen sowie rauer oder trockener Haut.

Eine Aufgabe der vorliegenden Erfindung war die Bereitstellung von Hautpflegezusammensetzungen zur nicht-therapeutischen Behandlung von Falten und Fältchen, die gegenüber dem Stand der Technik ein verbessertes Hautgefühl und ein verbessertes Hautbild liefern.

Eine weitere Aufgabe der vorliegenden Erfindung war die Bereitstellung von Hautpflegezusammensetzungen zur nicht-therapeutischen Behandlung von rauer oder trockener Haut, die gegenüber dem Stand der Technik ein verbessertes Hautgefühl und ein verbessertes Hautbild liefern.

Hydrogele sind wasserenthaltende Systeme auf Basis hydrophiler, aber wasserunlöslicher Polymere, die als dreidimensionales Netzwerk vorliegen. Die Bildung dieser Netzwerke erfolgt vorwiegend über kovalente Bindungen oder über elektrostatische, hydrophobe oder Dipol-Dipol-Wechselwirkungen.

Überraschend wurde nun gefunden, dass nanoskalige Hydrogele erfolgreich zur nicht-therapeutischen Behandlung von Falten und Fältchen sowie rauer oder trockener Haut eingesetzt werden können.

Ein erster Gegenstand der vorliegenden Erfindung ist daher die Verwendung von nanoskaligen Hydrogelen aus Polymeren mit einem mittleren Teilchendurchmesser von 10 - 400 nm und einer LCST (Lower Critical Solution Temperature, untere kritische Entmischungstemperatur) von 28 - 38 °C, ausgewählt aus Poly-N-isopropylacrylamiden, Polyvinylethern, Polyacrylaten, Polymethacrylaten, Polyacrylamiden, Polymethacrylamiden, Polyurethanen, Polyvinylpyrrolidonen und Polyvinylalkoholen sowie deren Copolymeren, alkylierten und/oder hydroxyalkylierten Polysacchariden und Celluloseethern, in einem zur topischen Anwendung geeigneten Träger mit einem Feststoffgehalt an LCST-Polymer von 0,5-1,5 Gew.-% zur nicht-therapeutischen Behandlung von Falten und Fältchen, rauer Haut und/oder trockener Haut.

Für den Einsatz in Form von nanoskaligen Hydrogelen sind natürliche Polymere geeignet, z. B. Agarose, Gelatine, Curdlan, Alginate, Pektinate, Carragheenane und Chitosane. Ebenfalls geeignet und erfindungsgemäß bevorzugt sind temperatursensitive Polymere mit LCST-Charakteristik (Lower Critical Solution Temperature, untere kritische Entmischungstemperatur). Insbesondere sind solche temperatursensitiven Polymere geeignet, die eine LCST im Bereich der Körper- bzw. Hauttemperatur, also etwa 28 - 38 °C, aufweisen. Solche temperatursensitiven Polymere sind bevorzugt erhältlich auf der Basis von Poly-N-isopropylacrylamid, das eine LCST von 30 - 32 °C aufweist. Andere Polymere mit einer ähnlichen LCST sind z. B. Polyvinylether (LCST ca. 33 °C), weiterhin Polyacrylate, Polymethacrylate, Polyacrylamide, Polymethacrylamide, Polyurethane, Polyvinylpyrrolidone und Polyvinylalkohole sowie deren Copolymere. Durch Copolymerisation mit hydrophoben Comonomeren lässt sich die LCST erhöhen, durch hydrophile Comonomere erniedrigen. Polyacrylate und insbesondere Copolymere mit Dimethylaminoethylmethacrylat und Ethylenglycoldimethacrylat weisen ebenfalls eine durch den Copolymerisationgrad individuell einstellbare LCST-Charakteristik in dem für die Applikation auf der Haut relevanten Bereich von 30 - 38 °C auf.

Weitere geeignete Polymere mit LCST-Charakteristik sind ausgewählt aus alkylierten und/oder hydroxyalkylierten Polysacchariden und Celluloseethern.

Beispiele für alkylierte und/oder hydroxyalkylierte Polysaccharide sind Methylhydroxypropylmethylcellulose (MHPC), Ethyl(hydroxyethyl)cellulose (EHEC), Hydroxypropylcellulose (HPC), Methylcellulose (MC), Ethylcellulose (EC), Carboxymethylcellulose (CMC), Carboxymethylmethylcellulose (CMMC), Hydroxybutylcellulose (HBC), Hydroxybutylmethylcellulose (HBMC), Hydroxyethylcellulose (HEC), Hydroxyethylcarboxymethylcellulose (HECMC), Hydroxyethylethylcellulose (HEEC), Hydroxypropylcellulose (HPC), Hydroxypropylcarboxymethylcellulose (HPCMC), Hydroxyethylmethylcellulose (HEMC), Methylhydroxyethylcellulose (MHEC), Methylhydroxyethylpropylcellulose (MHEPC), Methylcellulose (MC) und Propylcellulose (PC) und deren Gemische, wobei Carboxymethylcellulose, Methylcellulose, Methylhydroxyethylcellulose und Methylhydroxypropylcellulose sowie die Alkalisalze der CMC und die leicht ethoxylierte MC oder Gemische der voranstehenden bevorzugt sind. Weitere Beispiele für LCST-Substanzen sind Celluloseether sowie Gemische von Celluloseethern mit Carboxymethylcellulose (CMC).

Weitere Polymere, die eine untere kritische Entmischungstemperatur in Wasser zeigen und die ebenfalls erfindungsgemäß geeignet sind, sind Polymere von Mono- oder Di-N-alkylierten Acrylamiden, Copolymere von Mono- oder Di-N-substituierten Acrylamiden mit Acrylaten und/oder Acrylsäuren oder Gemische von miteinander verschlungenen Netzwerken der oben genannten (Co)Polymere. Geeignet sind außerdem Polyethylenoxid oder Copolymere davon, wie Ethylenoxid/Propylenoxidcopolymere und Pfropfcopolymere von alkylierten Acrylamiden mit Polyethylenoxid, Polymethacrylsäure, Polyvinylalkohol und Copolymere davon, Polyvinylmethylether, Poly(VATGVV), eine sich wiederholende Einheit in dem natürlichen Protein Elastin, und Alginate. Gemische aus diesen Polymeren mit Salzen oder Tensiden können ebenfalls als LCST-Substanz verwendet werden. Durch derartige Zusätze oder durch den Vernetzungsgrad der Polymere kann die LCST (untere kritische Entmischungstemperatur) entsprechend modifiziert werden.

Kosmetische Zusammensetzungen, die Polymere mit einer LCST-Charakteristik enthalten, sind im Stand der Technik bekannt. Die Offenlegungsschrift WO 98/48768 offenbart ein "reverse thermal viscosifying" Polymernetzwerk, enthaltend statistische Ethylenoxid-Propylenoxid-Ethylenoxid-Blockcopolymere, an die Acrylsäure anpolymerisiert ist. Das Polymernetzwerk wirkt bei Temperaturerhöhung verdickend und kann in kosmetischen Zusammensetzungen eingesetzt werden.

Die Offenlegungsschrift WO 00/07603 offenbart "reverse thermal viscosifying" Hydrogele, enthaltend ein Blockcopolymer aus einem Ethylenoxid-Propylenoxid-Block und einem Block aus Acrylsäure, Methacrylsäure oder deren Derivaten, sowie die Verwendung derartiger Hydrogele in kosmetischen Zusammensetzungen.

Die Offenlegungsschrift JP 08003019 offenbart puderförmige kosmetische Zusammensetzungen, die ein Polymer, bevorzugt ein Polymer ausgewählt aus N-Isopropylacrylamid, N-Isopropylmethacrylamid, N-Pentylacrylamid, N-Pentylmethacrylamid, N-Hexylacrylamid und N-Cyclohexylmethacrylamid, enthalten. Das Polymer dient insbesondere zur Verbesserung der Abriebfestigkeit von Make-up-Zusammensetzungen.

Die Patentschrift US 6,159,457 offenbart kosmetische Zusammensetzungen auf der Basis von wässrig-organischen Lösungen, die ein nicht-vernetztes Polymer mit LCST-Charakteristik enthalten und zur Filmbildung auf keratinischen Materialien verwendet werden. Die offenbarten Zusammensetzungen sind insbesondere zum Haar-Styling oder für Make-up-Zusammensetzungen auf wässriger Basis geeignet.

Die Offenlegungsschrift WO 95/24430 offenbart Pfropf-Copolymere mit einer LCST von 20 - 40 °C, die eine Hauptkette aus einem pH-sensitiven Polymer mit Temperatur-sensitiven Seitenketten enthalten, sowie deren Verwendung in Flüssigkeiten, Gelen und Salben zur Applikation von pharmazeutischen Wirkstoffen.

WO 02/076392 A1 offenbart thermoreversible Gele auf der Basis von LCST-Blockcopolymeren zur Wirkstofffreisetzung aus pharmazeutischen Zusammensetzungen.

US 2002/187173 A1 offenbart die Verwendung von LCST-Polymeren mit einer unteren kritischen Entmischungstemperatur von 5 - 40°C zur Verbesserung der Haftfähigkeit kosmetischer Zusammensetzungen auf der Haut oder anderen keratinischen Substanzen.

Im Unterschied zum Stand der Technik werden die erfindungsgemäß verwendeten Hydrogele als nanoskalige Hydrogeldispersionen konfektioniert.

Die Herstellung mikro- und nanoskaliger Hydrogeldispersionen ist im Stand der Technik bekannt. Nanoskalige Hydrogelpartikel sind erhältlich über Mikroemulsionspolymerisation einer mit Emulgatoren stabilisierten Wasser-Öl-Emulsion durch Homogenisierung mittels Hochdruckhomogenisatoren oder Rotor-Stator-Homogenisatoren. Die wässrige Phase enthält dabei die gelösten Monomere. Die Zugabe hydrophober Initiatoren bei gleichzeitiger Anwesenheit bi- bzw. multifunktionaler Monomere führt zur Ausbildung des polymeren Netzwerkes über kovalente Bindungen. Die Monomere können auch durch die Zugabe von Tensiden zu einer wässrigen Lösung in Emulsion gehalten werden; die Polymerisation erfolgt dann in den gebildeten Micellen. Die Hydrogele können weiterhin aus direkt aus Polymeren hergestellt werden, indem diese mikroemulgiert werden. Durch Temperatursenkung der Polymer-Mikroemulsion oder -Emulsion gelieren die Polymere, z. B. Gelatine, Agarose oder Polyvinylalkohol, wenn sie bis unter den Gefrierpunkt abgekühlt und langsam wieder aufgetaut werden.

Erfindungsgemäß sind Hydrogele geeignet, deren mittlerer Teilchendurchmesser 10 - 400 nm und besonders bevorzugt 50 - 400 nm beträgt. Erfindungsgemäß besonders bevorzugt sind temperatursensitive Hydrogele, die eine Lower Critical Solution Temperature (LCST, "untere kritische Entmischungstemperatur") im Bereich von 28 - 38 °C, bevorzugt im Bereich von 30 - 34 °C, aufweisen. Diese Hydrogele liegen bei Raumtemperatur (etwa 20 °C) im gequollenen Zustand vor und kollabieren bei Erreichen der LCST, so dass spontan Wasser an die Umgebung abgegeben wird und das Polymer verdickt bzw. ausfällt.

Weiterhin wurde überraschend gefunden, dass die nanoskaligen Hydrogele aus mindestens einem Polymer mit einem mittleren Teilchendurchmesser von 10 - 1000 400 nm, das eine LCST (Lower Critical Solution Temperature, untere kritische Entmischungstemperatur) von 28 - 38 °C aufweist, in der Lage sind, die günstigen Eigenschaften, die bestimmte kosmetische Wirkstoffe, insbesondere ausgewählte verdickende Polymere, ausgewählte Siliconverbindungen, Effekt- oder Lichtschutzpigmente, Proteinhydrolysate, Mono-, Oligo- oder Polysaccharide und α-Hydroxycarbonsäuren oder α-Ketocarbonsäuren, auf das Hauterscheinungsbild insbesondere der rauen, trockenen oder Altershaut und/oder die Hautfeuchte ausüben, in für den Fachmann nicht vorauszusehender synergistischer Weise zu verstärken.

Ein weiterer Gegenstand der vorliegenden Erfindung sind kosmetische Zusammensetzungen, die in einem zur topischen Anwendung geeigneten Träger 0,5 - 1,5 Gew.-% mindestens eines nanoskaligen Hydrogels aus mindestens einem Polymer mit einem mittleren Teilchendurchmesser von 10 - 400 nm, das eine LCST (Lower Critical Solution Temperature, untere kritische Entmischungstemperatur) von 28 - 38 °C aufweist, und ausgewählt ist aus Poly-N-isopropylacrylamiden, Polyvinylethern, Polyacrylaten, Polymethacrylaten, Polyacrylamiden, Polymethacrylamiden, Polyurethanen, Polyvinylpyrrolidonen und Polyvinylalkoholen sowie deren Copolymeren, alkylierten und/oder hydroxyalkylierten Polysacchariden und Celluloseethern, sowie weiterhin mindestens ein verdickendes Polymer enthalten, das ausgewählt ist aus teilweise oder vollständig neutralisierten, gegebenenfalls vernetzten Polymeren aus mindestens einem der Monomere Acrylsäure, Methacrylsäure, Itaconsäure, Maleinsäure und 2-Methyl-2[(1-oxo-2-propenyl)-amino]-1-propansulfonsäure, das mindestens ein neutrales Comonomer, ausgewählt aus Vinylpyrrolidon, Acrylamid und den C₁-C₃₀-Estern von Acrylsäure, Methacrylsäure, Itaconsäure und Maleinsäure, enthalten kann. Diese erfindungsgemäße Wirkstoffkombination erlaubt die Formulierung lager- und temperaturstabiler kosmetischer Zusammensetzungen mit einem außergewöhnlich angenehmen sensorischen, insbesondere taktil-sensorischen, Eindruck sowie die Formulierung von Zusammensetzungen mit hervorragenden pflegenden und konditionierenden Eigenschaften und einem günstigen Umweltverhalten.

Ein besonders geeignetes Copolymer ist das Handelsprodukt Aristoflex^{®} AVC (ex Clariant), das in Form des Ammoniumsalzes als weißes Pulver vorliegt. Das vernetzte Copolymer aus Vinylpyrrolidon und 2-Methyl-2[(1-oxo-2-propenyl)-amino]-1-propansulfonsäure ist in den erfindungsgemäßen Zusammensetzungen zu 0,1 - 2,5 Gew.-%, bevorzugt 0,2 - 2,0 Gew.-% und besonders bevorzugt 0,5 - 1,5 Gew.-%, jeweils bezogen auf die gesamte Zusammensetzung, enthalten.

Weitere für die erfindungsgemäßen Zusammensetzungen geeignete Polymere sind ausgewählt aus anionischen Homo- und Copolymeren aus unvernetzten und vernetzten Polyacrylsäuren, die teilweise oder vollständig neutralisiert sind. Besonders bevorzugte anionische Homo- und Copolymere sind hierbei vernetzte Polyacrylsäuren, die teilweise oder vollständig neutralisiert sind. Solche Polymere sind zum Beispiel die Handelsprodukte der Carbopol^{®}-Serie, wie Carbopol^{®} 934, 940, 941, Carbopol^{®} Ultrez 10, Carbopol^{®} ETD 2001 und 2050 sowie die Produkte Tego Carbomer 140 und 141 und Synthalen^{®} L.

Ein weiteres, bevorzugtes anionisches Copolymer enthält als Monomer zu 80 - 98 % eine ungesättigte, gewünschtenfalls substituierte C₃₋₆-Carbonsäure oder ihr Anhydrid sowie zu 2 - 20 % gewünschtenfalls substituierte Acrylsäureester von gesättigten C₁₀₋₃₀-Carbonsäuren, wobei das Copolymer mit den vorgenannten Vernetzungsagentien vernetzt sein kann. Entsprechende Handelsprodukte sind beispielsweise Pemulen^{®} und die Carbopol^{®}-Typen 954, 980, 1342 und ETD 2020 (ex B. F. Goodrich).

Weitere besonders bevorzugte Polymere sind teilweise oder vollständig neutralisierte, vernetzte Copolymere aus 2-Methyl-2[(1-oxo-2-propenyl)amino]-1-propansulfonsäure (AMPS) und Hydroxyethylacrylat. In einer außerordentlich bevorzugten Ausführungsform der Erfindung werden diese Copolymere in Form eines inversen Latex eingesetzt, kommerziell z. B. unter dem Handelsnamen Simulgel^{®}NS von der Firma Seppic erhältlich.

Weiterhin besonders bevorzugt sind teilweise oder vollständig neutralisierte, vernetzte Copolymere aus 2-Methyl-2[(1-oxo-2-propenyl)amino]-1-propansulfonsäure (AMPS) und Acrylamid. In einer außerordentlich bevorzugten Ausführungsform der Erfindung werden diese Copolymere in Form eines inversen Latex eingesetzt, der z. B. unter dem Handelsnamen Simulgel^{®}600 von der Firma Seppic erhältlich ist.

Weiterhin besonders bevorzugt sind teilweise oder vollständig neutralisierte, vernetzte Copolymere aus 2-Methyl-2[(1-oxo-2-propenyl)amino]-1-propansulfonsäure (AMPS) und Acrylsäure. In einer außerordentlich bevorzugten Ausführungsform der Erfindung werden diese Copolymere in Form eines inversen Latex eingesetzt, der z. B. unter dem Handelsnamen Simulgel^{®}EG von der Firma Seppic erhältlich ist.

Allen vorgenannten, bevorzugt verwendeten inversen Polymerlatices ist gemein, dass sie mindestens ein Öl, ausgewählt aus weißen Mineralölen, Squalan und hydriertem Polyisobuten, sowie mindestens einen Öl-in-Wasser-Emulgator, ausgewählt aus den Ethylenoxid-Addukten von Sorbitanoleat, Ricinusöl, das gewünschtenfalls gehärtet ist, Sorbitanlaurat und Laurylalkohol, enthalten und einen Polymergehalt von 30 - 90 Gew.%, bevorzugt 50 - 80 Gew.% und besonders bevorzugt 60 - 75 Gew.%, jeweils bezogen auf den gesamten Latex, aufweisen.

Das verdickende Polymer wird erfindungsgemäß in Mengen von 0,1 - 5 Gew.%, bevorzugt 0,2 - 3 Gew.% und besonders bevorzugt 0,3 - 2,5 Gew.%, bezogen auf die gesamte kosmetische Zusammensetzung, eingesetzt.

Im Sinne der vorliegenden Erfindung sind alle Polyelektrolytmonomere, die mit einer schwachen oder starken Säuregruppe funktionalisiert sind, teilweise oder vollständig neutralisiert als Natrium-, Kalium-, Ammonium-, Ethanolamin- oder Aminosäure-Salz.

Ein weiterer Gegenstand der vorliegenden Erfindung sind kosmetische Zusammensetzungen, die in einem zur topischen Anwendung geeigneten Träger 0,5-1,5 Gew.-% mindestens eines nanoskaligen Hydrogels aus mindestens einem Polymer mit einem mittleren Teilchendurchmesser von 10 - 400 nm, das eine LCST (Lower Critical Solution Temperature, untere kritische Entmischungstemperatur) von 28 - 38 °C aufweist und ausgewählt ist aus Poly-N-isopropylacrylamiden, Polyvinylethern, Polyacrylaten, Polymethacrylaten, Polyacrylamiden, Polymethacrylamiden, Polyurethanen, Polyvinylpyrrolidonen und Polyvinylalkoholen sowie deren Copolymeren, alkylierten und/oder hydroxyalkylierten Polysacchariden und Celluloseethern, sowie weiterhin mindestens eine Siliconverbindung enthalten, die ausgewählt ist aus Decamethylcyclopentasiloxan, Dodecamethylcyclohexasiloxan und Siliconpolymeren, die gewünschtenfalls quervernetzt sein können, z. B. Polydialkylsiloxane, Polyalkylarylsiloxane, ethoxylierte Polydialkylsiloxane sowie Polydialkylsiloxane, die Amin- und/oder HydroxyGruppen enthalten, darunter insbesondere Silicon-Elastomere. Silicon-Elastomere sind erhältlich durch die Vernetzung eines Organopolysiloxans, mit mindestens 2 C₂-C₁₀-Alkenylgruppen mit terminaler Doppelbindung in jedem Molekül enthält, mit einem Organopolysiloxan, das mindestens 2 Silicon-gebundene Wasserstoffatome in jedem Molekül aufweist. Das Organopolysiloxan mit mindestens zwei C₂-C₁₀-Alkenyl-Gruppen mit terminaler Doppelbindung im Molekül ist ausgewählt aus Methylvinylsiloxanen, Methylvinylsiloxan-Dimethylsiloxan-Copolymeren, Dimethylpolysiloxanen mit Dimethylvinylsiloxy-Endgruppen, Dimethylsiloxan-Methylphenylsiloxan-Copolymeren mit Dimethylvinylsiloxy-Endgruppen, Dimethylsiloxan-Diphenylsiloxan-Methylvinylsiloxan-Copolymeren mit Dimethylvinylsiloxy-Endgruppen, Dimethylsiloxan-Methylvinylsiloxan-Copolymeren mit Trimethylsiloxy-Endgruppen, Dimethylsiloxan-Methylphenylsiloxan-Methylvinylsiloxan-Copolymeren mit Trimethylsiloxy-Endgruppen, Methyl(3,3,3-Trifluoropropyl)Polysiloxanen mit Dimethylvinylsiloxy-Endgruppen und Dimethylsiloxan-Methyl(3,3,3-Trifluoropropyl)Siloxan-Copolymeren mit Dimethylvinylsiloxy-Endgruppen.

Das vernetzende Organopolysiloxan mit mindestens zwei Silicon-gebundenen Wasserstoffatomen ist ausgewählt aus Methylhydrogenpolysiloxanen mit Trimethylsiloxy-Endgruppen, Dimethylsiloxan-Methylhydrogensiloxan-Copolymeren mit Trimethylsiloxy-Endgruppen und cyclischen Dimethylsiloxan-Methylhydrogen-Siloxan-Copolymeren.

Die erfindungsgemäßen Zusammensetzungen können sowohl nicht-emulgierende als auch emulgierende Silicon-Elastomere sowie deren Mischungen enthalten.

Geeignete nicht-emulgierende Silicon-Elastomere sind im Handel erhältlich, beispielsweise von Dow Corning die Produkte DC 9040, DC 9041 oder DC 9509, von General Electric, z. B. das Handelsprodukt SFE 839, von Shin-Etsu die Handelsprodukte KSG-15, KSG-16 oder KSG-18 sowie von Grant Industries die Produkte aus der Gransil^{®}-Serie, wie z. B. Gransil^{®}RPS Gel, INCI-Bezeichnung Cyclopentasiloxane and Polysilicone-11 oder Gransil^{®}GCM-4, INCI-Bezeichnung Cyclotetrasiloxane and Polysilicone-11.

Geeignete emulgierende Silicon-Elastomere enthalten als funktionelle Gruppen am Polysiloxan-Gerüst Polyoxyethylen- und/oder Polyoxypropylen-Gruppen. Diese Gruppen können endständig und/oder als Seitengruppen zur Polysiloxan-Kette angeordnet sein. Geeignete emulgierende Silicon-Elastomere sind im Handel erhältlich, beispielsweise von Shin-Etsu die Produkte KSG-21, KSG-31, KSG-31X, KSG-32 oder von Dow Corning das Handelsprodukt DC-9011.

Der Gehalt an nicht-emulgierendem und/oder emulgierendem Silicon-Elastomer beträgt in den erfindungsgemäßen Zusammensetzungen 0,1 - 40 Gew.%, bevorzugt 0,5 - 20 Gew.% und besonders bevorzugt 1 - 10 Gew.%, jeweils bezogen auf die gesamte kosmetische Zusammensetzung.

Ein weiterer Gegenstand der vorliegenden Erfindung sind kosmetische Zusammensetzungen, die in einem zur topischen Anwendung geeigneten Träger 0,5-1,5 Gew.-% mindestens eines nanoskaligen Hydrogels aus mindestens einem Polymer mit einem mittleren Teilchendurchmesser von 10 - 400 nm, das eine LCST (Lower Critical Solution Temperature, untere kritische Entmischungstemperatur) von 28 - 38 °C aufweist und ausgewählt ist aus Poly-N-isopropylacrylamiden, Polyvinylethern, Polyacrylaten, Polymethacrylaten, Polyacrylamiden, Polymethacrylamiden, Polyurethanen, Polyvinylpyrrolidonen und Polyvinylalkoholen sowie deren Copolymeren, alkylierten und/oder hydroxyalkylierten Polysacchariden und Celluloseethern, sowie weiterhin mindestens ein Effektpigment oder Lichtschutzpigment enthalten.

Unter Effektpigmenten sind erfindungsgemäß Pigmente zu verstehen, die durch ihre Brechungseigenschaften besondere optische Effekte hervorrufen. Effektpigmente verleihen der behandelten Oberfläche (Haut, Haar, Schleimhaut) Glanz- oder Glitzereffekte oder können durch diffuse Lichtstreuung Hautunebenheiten und Hautfältchen optisch kaschieren. Als besondere Ausführungsform der Effektpigmente sind erfindungsgemäß Interferenzpigmente bevorzugt.

Erfindungsgemäß besonders geeignete Effektpigmente sind beispielsweise Glimmerpartikel, die mit mindestens einem Metalloxid beschichtet sind. Neben Glimmer, einem Schichtsilikat, sind auch Kieselgel und andere SiO₂-Modifikationen als Träger geeignet. Ein häufig zur Beschichtung verwendetes Metalloxid ist beispielsweise Titandioxid, dem gewünschtenfalls Eisenoxid beigemischt sein kann. Über die Größe und die Form (z. B. sphärisch, ellipsoid, abgeflacht, eben, uneben) der Pigmentpartikel sowie über die Dicke der Oxidbeschichtung können die Reflexionseigenschaften beeinflusst werden. Auch durch die Art und die Menge von zugesetzten, gegebenenfalls farbigen Oxiden, z. B. Eisenoxid, lassen sich verschiedene optische Effekte einstellen. Auch andere Metalloxide können Effektpigmente bilden, z. B. Bismutoxychlorid (BiOCl) sowie die Oxide von beispielsweise Titan, insbesondere die TiO₂-Modifikationen Anatas und Rutil, Aluminium, Tantal, Niob, Zirkon und Hafnium. Auch mit Magnesiumfluorid (MgF₂) und Calciumfluorid (Flussspat, CaF₂) können Effektpigmente hergestellt werden.

Die erfindungsgemäßen Zusammensetzungen enthalten die Effektpigmente in Mengen von 0,1 - 20 Gew.%, bevorzugt 0,5 - 10 und besonders bevorzugt 1 - 5 Gew.%, jeweils bezogen auf die gesamte Zusammensetzung.

Bei den erfindungsgemäß bevorzugten anorganischen Lichtschutzpigmenten handelt es sich um feindisperse oder kolloiddisperse Metalloxide und Metallsalze, beispielsweise Titandioxid, Zinkoxid, Eisenoxid, Aluminiumoxid, Ceroxid, Zirkoniumoxid, Silicate (Talk) und Bariumsulfat. Die Partikel sollten dabei einen mittleren Durchmesser von weniger als 100 nm, vorzugsweise zwischen 5 und 50 nm und insbesondere zwischen 15 und 30 nm aufweisen, so genannte Nanopigmente. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z. B. Titandioxid T 805 (Degussa) oder Eusolex^{®} T2000 (Merck). Als hydrophobe Coatingmittel kommen dabei vor allem Silicone und dabei speziell Trialkoxyoctylsilane oder Simethicone in Frage. Die bevorzugten anorganischen Partikelsubstanzen sind hydrophil oder amphiphil. Besonders bevorzugt sind Titandioxid und Zinkoxid. Bevorzugterweise sind oberflächlich wasserabweisend behandelt ("hydrophobiert"). Beispiele hierfür sind mit Aluminiumstearat beschichtete Titandioxid-Pigmente (Handelsprodukt MT 100 T von der Firma Tayca), mit Dimethylpolysiloxan (Dimethicone) beschichtetes Zinkoxid, mit Dimethicone beschichtetes Bornitrid (Très BN^{®} UHP 1106 von Carborundum), mit einem Gemisch aus Dimethylpolysiloxan und Silicagel (Simethicone) und Aluminiumoxidhydrat (Alumina) beschichtetes Titandioxid (Eusolex^{®} T 2000 von Merck), mit Octylsilanol beschichtetes Titandioxid oder sphärische Polyalkylsesquioxan-Partikel (Aerosil^{®} R972 und Aerosil^{®} 200V von Degussa).

Bei den erfindungsgemäß bevorzugten organischen Lichtschutzpigmenten handelt es sich um bei Raumtemperatur kristallin vorliegende Substanzen, die in der Lage sind, ultraviolette Strahlen zu absorbieren und die aufgenommene Energie in Form längerwelliger Strahlung, z. B. Wärme, wieder abzugeben. Man unterscheidet UVA-Filter und UVB-Filter. Die UVA- und UVB-Filter können sowohl einzeln als auch in Mischungen eingesetzt werden. Der Einsatz von Filtermischungen ist erfindungsgemäß bevorzugt.

Die erfindungsgemäß geeigneten organischen UV-Filter sind ausgewählt aus den bei Raumtemperatur festen Derivaten von Dibenzoylmethan, Zimtsäureestern, Diphenylacrylsäureestern, Benzophenon, Campher, p-Aminobenzoesäureestern, o-Aminobenzoesäureestern, Salicylsäureestern, Benzimidazolen, 1,3,5-Triazinen, monomeren und oligomeren 4,4-Diarylbutadiencarbonsäureestern und -carbonsäureamiden, Ketotricyclo(5.2.1.0)decan, Benzalmalonsäureestern sowie beliebigen Mischungen der genannten Komponenten. Die organischen UV-Filter können öllöslich oder wasserlöslich sein. Erfindungsgemäß besonders bevorzugte öllösliche UV-Filter sind 1-(4-tert.-Butylphenyl)-3-(4'methoxyphenyl)propan-1,3-dion (Parsol^{®} 1789), 1-Phenyl-3-(4'-isopropylphenyl)-propan-1,3-dion, 3-(4'-Methylbenzyliden)-D,L-campher, 4-(Dimethylamino)-benzoesäure-2-ethylhexylester, 4-(Dimethylamino)benzoesäure-2-octylester, 4-(Dimethylamino)-benzoesäureamylester, 4-Methoxyzimtsäure-2-ethylhexylester, 4-Methoxyzimtsäurepropylester, 4-Methoxyzimtsäureisopentylester, 2-Cyano-3,3-phenylzimtsäure-2-ethylhexylester (Octocrylene), Salicylsäure-2-ethylhexylester, Salicylsäure-4-isopropylbenzylester, Salicylsäurehomomenthylester (3,3,5-Trimethyl-cyclohexylsalicylat), 2-Hydroxy-4-methoxybenzophenon, 2-Hydroxy-4-methoxy-4'-methylbenzophenon, 2,2'-Dihydroxy-4-methoxybenzophenon, 4-Methoxybenzmalonsäuredi-2-ethylhexylester, 2,4,6-Trianilino-(p-carbo-2'-ethyl-1'-hexyloxy)-1,3,5-triazin (Octyl Triazone) und Dioctyl Butamido Triazone (Uvasorb^{®} HEB) sowie beliebige Mischungen der genannten Komponenten.

Bevorzugte wasserlösliche UV-Filter sind 2-Phenylbenzimidazol-5-sulfonsäure und deren Alkali-, Erdalkali-, Ammonium-, Alkylammonium-, Alkanolammonium- und Glucammoniumsalze, Sulfonsäurederivate von Benzophenonen, vorzugsweise 2-Hydroxy-4-methoxybenzophenon-5-sulfonsäure und ihre Salze, Sulfonsäurederivate des 3-Benzylidencamphers, wie z. B. 4-(2-Oxo-3-bornylidenmethyl)benzolsulfonsäure und 2-Methyl-5-(2-oxo-3-bornyliden)sulfonsäure und deren Salze.

In den erfindungsgemäßen Zusammensetzungen sind die anorganischen und organischen Lichtschutzpigmente in Mengen von 0,1 - 30 Gew.-%, bevorzugt 1 - 20 Gew.% und besonders bevorzugt 2 - 15 Gew.-%, jeweils bezogen auf die gesamte Zusammensetzung, enthalten.

Ein weiterer Gegenstand der vorliegenden Erfindung sind kosmetische Zusammensetzungen, die in einem zur topischen Anwendung geeigneten Träger 0,5-1,5 Gew.-% mindestens eines nanoskaligen Hydrogels aus mindestens einem Polymer mit einem mittleren Teilchendurchmesser von 10 - 400 nm, das eine LCST (Lower Critical Solution Temperature, untere kritische Entmischungstemperatur) von 28 - 38 °C aufweist und ausgewählt ist aus Poly-N-isopropylacrylamiden, Polyvinylethern, Polyacrylaten, Polymethacrylaten, Polyacrylamiden, Polymethacrylamiden, Polyurethanen, Polyvinylpyrrolidonen und Polyvinylalkoholen sowie deren Copolymeren, alkylierten und/oder hydroxyalkylierten Polysacchariden und Celluloseethern, sowie weiterhin mindestens ein Proteinhydrolysat enthalten. Erfindungsgemäß können sowohl pflanzliche als auch tierische Proteinhydrolysate eingesetzt werden. Tierische Proteinhydrolysate sind z. B. Elastin-, Collagen-, Keratin-, Seiden- und Milcheiweiß-Proteinhydrolysate, die auch in Form von Salzen vorliegen können. Erfindungsgemäß bevorzugt sind pflanzliche Proteinhydrolysate, z. B. Soja-, Weizen-, Mandel-, Erbsen-, Kartoffel- und Reisproteinhydrolysate. Entsprechende Handelsprodukte sind z. B. DiaMin^{®} (Diamalt), Gluadin^{®} (Cognis), Lexein^{®} (Inolex) und Crotein^{®} (Croda).

Wenngleich der Einsatz der zusätzlichen Proteinhydrolysate als solche bevorzugt ist, können an ihrer Stelle gegebenenfalls auch anderweitig erhaltene Aminosäuregemische oder einzelne Aminosäuren wie beispielsweise Asparaginsäure, Arginin, Lysin, Histidin oder Pyroglutaminsäure sowie deren Salze eingesetzt werden. Ebenfalls möglich ist der Einsatz von Derivaten der Proteinhydrolysate, z. B. in Form ihrer Fettsäure-Kondensationsprodukte. Entsprechende Handelsprodukte sind z. B. Lamepon^{®} (Cognis), Gluadin^{®} (Cognis), Lexein^{®} (Inolex), Crolastin^{®} oder Crotein^{®} (Croda).

In den erfindungsgemäßen Zusammensetzungen sind die Proteinhydrolysate in Mengen von 0,01 - 10 Gew.-%, bevorzugt 0,1 - 5 Gew.% und besonders bevorzugt 0,2 - 3 Gew.-%, jeweils bezogen auf die gesamte Zusammensetzung, enthalten.

Ein weiterer Gegenstand der vorliegenden Erfindung sind kosmetische Zusammensetzungen, die in einem zur topischen Anwendung geeigneten Träger 0,5-1,5 Gew.-% mindestens eines nanoskaligen Hydrogels aus mindestens einem Polymer mit einem mittleren Teilchendurchmesser von 10 - 400 nm, das eine LCST (Lower Critical Solution Temperature, untere kritische Entmischungstemperatur) von 28 - 38 °C aufweist und ausgewählt ist aus Poly-N-isopropylacrylamiden, Polyvinylethern, Polyacrylaten, Polymethacrylaten, Polyacrylamiden, Polymethacrylamiden, Polyurethanen, Polyvinylpyrrolidonen und Polyvinylalkoholen sowie deren Copolymeren, alkylierten und/oder hydroxyalkylierten Polysacchariden und Celluloseethern, sowie weiterhin mindestens ein Mono-, Oligo- oder Polysaccharid enthalten. Erfindungsgemäß geeignete Monosaccharide sind z. B. Glucose, Fructose, Galactose, Arabinose, Ribose, Xylose, Lyxose, Allose, Altrose, Mannose, Gulose, Idose und Talose sowie die Desoxyzucker Fucose und Rhamnose. Bevorzugt sind Glucose, Fructose, Galactose, Arabinose und Fucose; Glucose ist besonders bevorzugt.

Erfindungsgemäß geeignete Oligosaccharide sind aus zwei bis zehn Monosaccharideinheiten zusammengesetzt, z. B. Saccharose, Lactose oder Trehalose. Ein besonders bevorzugtes Oligosaccharid ist Saccharose. Ebenfalls besonders bevorzugt ist die Verwendung von Honig, der überwiegend Glucose und Saccharose enthält.

Weiterhin können die erfindungsgemäßen Zusammensetzungen Polysaccharide mit mehr als zehn Monosaccharideinheiten enthalten. Bevorzugte Polysaccharide sind die aus α-D-Glucose-Einheiten aufgebauten Stärken sowie Stärkeabbauprodukte wie Amylose, Amylopektin und Dextrine. Erfindungsgemäß besonders vorteilhaft sind chemisch und/oder thermisch modifizierte Stärken, z. B. Hydroxypropylstärkephosphat, Dihydroxypropyldistärkephosphat oder die Handelsprodukte Dry Flo^{®}. Weiterhin bevorzugt sind Dextrane sowie ihre Derivate, z. B. Dextransulfat. Ebenfalls bevorzugt sind nichtionische Cellulose-Derivate, wie Methylcellulose, Hydroxypropylcellulose, Hydroxypropylmethylcellulose oder Hydroxyethylcellulose, sowie kationische Cellulose-Derivate, z. B. die Handelsprodukte Celquat^{®} und Polymer JR^{®}, und bevorzugt Celquat^{®} H 100, Celquat^{®} L 200 und Polymer JR^{®} 400 (Polyquaternium-10) sowie Polyquaternium-24. Weitere bevorzugte Beispiele sind Polysaccharide mit Fucose- und/oder Galactose- und/oder Galacturonsäure-Einheiten, z. B. das Handelsprodukt Fucogel^{®}. In den erfindungsgemäßen Zusammensetzungen sind die Mono-, Oligo- oder Polysaccharide oder deren Derivate in Mengen von 0,1 - 10 Gew.-%, bevorzugt 0,5 - 5 Gew.% und besonders bevorzugt 1,0 - 3 Gew.-%, jeweils bezogen auf die gesamte Zusammensetzung, enthalten.

Ein weiterer Gegenstand der vorliegenden Erfindung sind kosmetische Zusammensetzungen, die in einem zur topischen Anwendung geeigneten Träger 0,5-1,5 Gew.-% mindestens eines nanoskaligen Hydrogels aus mindestens einem Polymer mit einem mittleren Teilchendurchmesser von 10 - 400 nm, das eine LCST (Lower Critical Solution Temperature, untere kritische Entmischungstemperatur) von 28 - 38 °C aufweist und ausgewählt ist aus Poly-N-isopropylacrylamiden, Polyvinylethern, Polyacrylaten, Polymethacrylaten, Polyacrylamiden, Polymethacrylamiden, Polyurethanen, Polyvinylpyrrolidonen und Polyvinylalkoholen sowie deren Copolymeren, alkylierten und/oder hydroxyalkylierten Polysacchariden und Celluloseethern, sowie weiterhin mindestens eine α-Hydroxycarbonsäure oder α-Ketocarbonsäure oder deren Ester-, Lacton- oder Salzform enthalten. Geeignete α-Hydroxycarbonsäuren oder α-Ketocarbonsäuren sind ausgewählt aus Milchsäure, Weinsäure, Citronensäure, 2-Hydroxybutansäure, 2,3-Dihydroxypropansäure, 2-Hydroxypentansäure, 2-Hydroxyhexansäure, 2-Hydroxyheptansäure, 2-Hydroxyoctansäure, 2-Hydroxydecansäure, 2-Hydroxydodecansäure, 2-Hydroxytetradecansäure, 2-Hydroxyhexadecansäure, 2-Hydroxyoctadecansäure, Mandelsäure, 4-Hydroxymandelsäure, Äpfelsäure, Erythrarsäure, Threarsäure, Glucarsäure, Galactarsäure, Mannarsäure, Gularsäure, 2-Hydroxy-2-methylbernsteinsäure, Gluconsäure, Brenztraubensäure, Glucuronsäure und Galacturonsäure. Die Ester der genannten Säuren sind ausgewählt aus den Methyl-, Ethyl-, Propyl-, Isopropyl-, Butyl-, Amyl-, Pentyl-, Hexyl-, 2-Ethylhexyl-, Octyl-, Decyl-, Dodecyl- und Hexadecylestern. Die α-Hydroxycarbonsäuren oder α-Ketocarbonsäuren oder ihre Derivate sind in Mengen von 0,1 - 10 Gew.-%, bevorzugt 0,5 - 5 Gew.-%, jeweils bezogen auf die gesamte Zusammensetzung, enthalten.

Vorteilhafterweise liegen die erfindungsgemäßen kosmetischen Zusammensetzungen in Form einer flüssigen oder festen Öl-in-Wasser-Emulsion, Wasser-in-Öl-Emulsion, Mehrfach-Emulsion, Mikroemulsion, PIT-Emulsion oder Pickering-Emulsion, eines Hydrogels, einer ein- oder mehrphasigen Lösung oder eines Schaumes vor.

In einer besonderen Ausführungsform der erfindungsgemäßen Mittel liegen die Mittel als Mikroemulsion vor. Unter Mikroemulsionen werden im Rahmen der Erfindung neben den thermodynamisch stabilen Mikroemulsionen auch die sogenannten "PIT"-Emulsionen verstanden. Bei diesen Emulsionen handelt es sich um Systeme mit den drei Komponenten Wasser, Öl und Emulgator, die bei Raumtemperatur als Öl-in-Wasser-Emulsion vorliegen. Beim Erwärmen dieser Systeme bilden sich in einem bestimmten Temperaturbereich (als Phaseninversiontemperatur oder "PIT" bezeichnet) Mikroemulsionen aus, die sich bei weiterer Erwärmung in Wasser-in-ÖI-Emulsionen umwandeln. Bei anschließendem Abkühlen werden wieder O/W-Emulsionen gebildet, die aber auch bei Raumtemperatur als Mikroemulsionen oder als sehr feinteilige Emulsionen mit einem mittleren Teilchendurchmesser unter 400 nm und insbesondere von etwa 100-300 nm, vorliegen. Erfindungsgemäß können solche Mikro- oder "PIT"-Emulsionen bevorzugt sein, die einen mittleren Teilchendurchmesser von etwa 200 nm aufweisen. Ebenfalls fallen erfindungsgemäß unter den Begriff "Mikroemulsion" solche feinstteiligen Emulsionen, die mittels Hochdruckhomogenisierung erhältlich sind, ein teilchengröße von 10 - 300 nm, bevorzugt 50 - 300 nm, besonders bevorzugt 50 - 150 nm und außerordentlich bevorzugt 50 - 100 nm, aufweisen. Für derartige Emulsionen findet sich in der Literatur auch der Begriff "Nanoemulsion".

Selbstverständlich lassen sich die erfindungsgemäßen Zusammensetzungen je nach Anwendungsbereich, z. B. als Reinigungsmittel, optional auch mit Emulgatoren, Dispergiermitteln oder schäumenden Tensiden formulieren, die durchschnittlich mehr als 10 Alkylenoxid-Gruppen pro Molekül aufweisen. Geeignete Emulgatoren sind beispielsweise Anlagerungsprodukte von 11 bis 30 Mol Ethylenoxid und ggf. 0 - 5 Mol Propylenoxid an lineare C₈-C₂₂-Fettalkohole, an C₁₂-C₂₂-Fettsäuren und an C₈-C₁₅-Alkylphenole, Anlagerungsprodukte von 11 - 30 Mol Ethylenoxid an Methylglucosid-Fettsäureester, Fettsäurealkanolamide, Fettsäureglucamide und C₈-C₂₂-Alkylmono- und - oligoglycoside, wobei Oligomerisierungsgrade von 1,1 bis 5, insbesondere 1,2 bis 2,0, und Glucose als Zuckerkomponente bevorzugt sind.

Die erfindungsgemäßen Mittel enthalten die optionalen Emulgatoren bevorzugt in Mengen von 0,1 bis 25 Gew.-%, insbesondere 0,5 - 15 Gew.-%, bezogen auf das gesamte Mittel.

Die erfindungsgemäßen Mittel können weitere Wirk-, Hilfs- und Zusatzstoffe enthalten, beispielsweise Vitamine, Provitamine und Vitaminvorstufen aus den Gruppen A, B (insbesondere Panthenol und Pantolacton), C, E und F, Allantoin, Bisabolol und Antioxidantien.

Folgende Beispiele sollen die Erfindung verdeutlichen, ohne sie hierauf zu beschränken.

### Beispiele

### Herstellung der Poly-N-isopropylacrylamid-Hydrogeldispersion

In einem 1-I-Vierhalskolben wurden 470 ml vollentsalztes Wasser, 7 g N-Isopropylacrylamid, 0,35 g N,N-Methylenbisacrylamid und 0,094 g Natriumdodecylsulfat eingewogen und unter Stickstoffatmosphäre, Rückfluss und Rühren bei 200 UpM auf 70 °C erwärmt. Nach 30 Minuten wurden 0,28 g Kaliumperoxodisulfat, in 30 ml vollentsalztem Wasser gelöst, hinzugegeben. Die Reaktion wurde nach weiteren 4 Stunden beendet. Durch anschließende Dialyse wurde eine wässrige Hydrogeldispersion erhalten.

Die so hergestellten Hydrogelpartikel wiesen bei Raumtemperatur (ca. 20 °C) einen durchschnittlichen Teilchendurchmesser von 200 - 300 nm auf. Bei Erreichen der LCST nahm der Partikeldurchmesser auf Werte von 100 - 200 nm ab.

Die Menge der eingesetzten Chemikalien konnte soweit erhöht werden, dass die Konzentration an Hydrogelpartikeln in der wässrigen Dispersion nicht, wie im obigen Beispiel, 1,4 Gew.% betrug, sondern auf Konzentrationen von mehr als 5 Gew.% erhöht werden konnte, ohne dass die Partikelgrößenverteilung nachteilig beeinflusst wurde.

### Herstellung der nanoskaligen Hydrogelmatrix A

Durch Abtrennung des Wasser konnte aus der oben dargestellten 5 Gew.%-igen Hydrogeldispersion ein Gel erhalten werden. Hierzu wurde die Hydrogeldispersion gemäß dem obenstehenden Beispiel in einem offenen Gefäß in einem auf 90 °C temperierten Trockenschrank soweit eingeengt, bis das Gel als unlösliche Phase ausfiel. Die wässrige Phase konnte vom Gelkörper dekantiert werden. Alternativ ließen sich die Hydrogelpartikel durch Zentrifugation bei Umdrehungszahlen von mehr als 100 000 UpM abtrennen.

Eine vollständige Trocknung der Hydrogelpartikel wurde durch Gefriertrocknung erzielt. Der hierbei erhaltene Feststoff kann in einer definierten Menge Wasser aufgequollen werden.

### Herstellung einer kosmetischen Zusammensetzung und Applikation auf der Haut

Aus dem mittels Gefriertrocknung erhaltenen Feststoff wurde eine 20 Gew.%ige wässrige Dispersion hergestellt und mehrere Stunden zur Quellung stehen gelassen.

Auf diese Weise wurde ein Gel mit einem Gehalt an Poly-N-isopropylacrylamid von 20 Gew.% und einem Wassergehalt von 80 Gew.% hergestellt.

Die Applikation sowohl dieses Gels als auch der nachstehend beispielhaft aufgeführten kosmetischen Zusammensetzungen, die ein erfindungsgemäßes Gel enthielten, auf die Haut führte zu einer signifikanten Verringerung der Faltentiefe.

Eine Applikation auf raue Haut führte zu einer signifikanten Verbesserung des Hauterscheinungsbildes; die Haut fühlte sich geschmeidiger, weicher und glatter an. Ebenso wirkte die Haarstruktur in dem behandelten Bereich weicher, das heißt, die Haare wurden nicht mehr so deutlich wahrgenommen. Diese Effekte hielten mehrere Tage lang an und wurden auch durch Waschen nicht verändert.

Die Applikation auf trockene Haut führte zu einer signifikanten Verbesserung der Hautfeuchtigkeit, die auch noch mehrere Stunden nach der Applikation nachweisbar war.

Die kosmetischen Zusammensetzungen können im Polymergehalt variieren. Geeignet sind Feststoffgehalte von 0,5 - 50 Gew.%, bevorzugt 1,0 - 30 Gew.% und besonders bevorzugt 1,5 - 10 Gew.%, um eine gute Verteilbarkeit auf der Haut zu gewährleisten.

Die erfindungsgemäß verwendeten Zusammensetzungen können weiterhin übliche kosmetische Wirk- und Hilfsstoffe, wie Öle, Fette, Emulgatoren, Vitamine, UV-Filter, Konservierungsmittel, Antioxidantien, Pflanzenextrakte und dergleichen, enthalten.

**Beispielrezepturen (Mengenangaben in Gewichts-%)**

| | | **1.1** | **1.2** | **1.3** |
|---|---|---|---|---|
| Phase 1 | Distelöl | 3,0 | 3,0 | 3,0 |
| | Myritol^{®} PC | 3,5 | 3,5 | 3,5 |
| | Lanette^{®} 22 | 3,0 | 3,0 | 3,0 |
| | Cutina^{®} GMS-V | 3,0 | 3,0 | 3,0 |
| | Stenol^{®} 16/18 | 2,0 | 2,0 | 2,0 |
| | Isopropylstearat | 6,0 | 6,0 | 6,0 |
| | Baysilon^{®} M350 | 1,0 | 1,0 | 1,0 |
| | Dow Corning^{®} 9040 | 0,5 | - | - |
| | Dow Corning^{®} 593 | - | 0,5 | - |
| | Controx^{®} KS | 0,05 | 0,05 | 0,05 |
| | Parsol^{®} 1789 | 2,0 | 2,0 | - |
| | Parsol^{®} MCX | 3,0 | 3,0 | - |
| | Eusolex^{®} 4360 | - | - | 0,5 |
| | Eusolex^{®} 6300 | 3,0 | 3,0 | 2,0 |
| | Uvinul^{®} T 150 | 2,5 | 2,5 | - |
| | Propylparaben | 0,2 | 0,2 | 0,2 |
| Phase 2 | Glycerin | 5,0 | 5,0 | 5,0 |
| | Trehalose | - | 1,0 | - |
| | Honig | 0,5 | - | - |
| | Methylparaben | 0,2 | 0,2 | 0,2 |
| | Wasser | 40,0 | 40,0 | 40,0 |
| Phase 3 | V-Protein flüssig (COS 152/22-A) | - | 9,0 | 9,0 |
| | Hibiscin^{®} HP-LS-9198 | 3,0 | - | - |
| Phase 4 | Granlux^{®} MSN 50 | 2,5 | 2,5 | - |
| Phase 5 | Citronensäure | 0,1 | 0,1 | 0,1 |
| Phase 6 | Lipochroman-6 | 0,01 | 0,01 | 0,01 |
| | Retinol | 0,1 | - | - |
| | Na-Ascorbylphosphat | - | 0,1 | - |
| | Mg-Aspartat | 0,2 | - | - |
| Phase 7 | Sepigel^{®} 305 | 2,0 | - | - |
| | Aristoflex^{®} AVC | - | 1,5 | - |
| | Simulgel^{®} NS | - | - | 2,0 |
| | * | 5,0 | - | - |
| | ** | - | 5,0 | - |
| | *** | - | - | 5,0 |
| | Wasser | ad 100 | ad 100 | ad 100 |

| | | | | |
|---|---|---|---|---|
| * 20%ige wässrige Dispersion der oben hergestellten nanoskaligen Hydrogelmatrix A ** 20%ige wässrige Dispersion einer nanoskaligen Hydrogelmatrix aus Hydroxypropylmethylcellulose *** 20%ige wässrige Dispersion einer nanoskaligen Hydrogelmatrix aus Polymethacrylat | | | | |

Die Mengenangaben in der vorstehenden Beispieltabelle sowie in den übrigen Tabellen beziehen sich bei Reinsubstanzen auf die Aktivsubstanz, bei Komponentengemischen auf die Menge des Rohstoffs tel quel.

### Herstellung:

Fett- und Wasserphase (Phase 1 und Phase 2) wurden getrennt voneinander auf etwa 80° C erhitzt. Die Wasserphase wurde unter dem Homogenisator vorgelegt. Dann wurden Phase 3 und Phase 4 zugegeben. Anschließend wurde etwa 1 Minute lang homogenisiert. Dann wurde die Fettphase hinzugegeben und erneut etwa 1 Minute lang homogenisiert. Nach dem Abkühlen auf etwa 30° C wurden Lipochroman-6 und die Vitamin-Wirkstoffe zugegeben und der pH-Wert mit Citronensäure auf einen Wert im Bereich von 4,8 - 5,2 eingestellt. Anschließend wurden das verdickende Polymer (Sepigel^{®} 305, Aristoflex^{®} AVC oder Simulgel^{®} NS) und das erfindungsgemäße nanoskalige Hydrogel hinzugegeben. Es bildete sich eine stabile Öl-in-Wasser-Emulsion, nach dem Abkühlen auf 20° C eine glatte Creme von hoher Stabilität.

Die Applikation der Zusammensetzungen auf die Haut führte zu einer signifikanten Verringerung der Faltentiefe.

Eine Applikation der Zusammensetzungen auf raue Haut führte zu eine signifikanten Verbesserung des Hauterscheinungsbildes; die Haut fühlte sich geschmeidiger, weicher und glatter an.

Die Applikation der Zusammensetzungen auf trockene Haut führte zu einer signifikanten Verbesserung der Hautfeuchtigkeit, die auch noch mehrere Stunden nach der Applikation nachweisbar war.

| **Komponente** | **2.2** | **2.3** |
|---|---|---|
| Hostaphat KL 340 N | 1,5 | 1,5 |
| Ethylhexylpalmitat | 3,0 | 3,0 |
| Paraffinöl | 8,0 | 8,0 |
| Tocopherylacetat | 0,01 | 0,01 |
| Lipochroman-6^{®} | 0,01 | 0,01 |
| Carbomer | 0,2 | 0,2 |
| NaOH | ad pH 4,8 - 5,2 | ad pH 4,8 - 5,2 |
| Propylenglycol | 10,0 | 10,0 |
| Methylparaben | 0,5 | 0,5 |
| Propylparaben | 0,5 | 0,5 |
| α-Bisabolol | - | - |
| α-Liponsäure | 0,05 | - |
| Citronensäure | - | - |
| Milchsäure | 0,5 | - |
| Gluconolacton | - | 0,5 |
| * | - | - |
| ** | 5,0 | |
| *** | - | 5,0 |
| Wasser | ad 100 | ad 100 |

| | | |
|---|---|---|
| * 20%ige wässrige Dispersion der oben hergestellten nanoskaligen Hydrogelmatrix A ** 20%ige wässrige Dispersion einer nanoskaligen Hydrogelmatrix aus Hydroxypropylmethylcellulose *** 20%ige wässrige Dispersion einer nanoskaligen Hydrogelmatrix aus Polymethacrylat | | |

### Herstellung:

Fett- und Wasserphase wurden getrennt voneinander auf etwa 80° C erhitzt. Die Wasserphase wurde unter dem Homogenisator vorgelegt. Dann wurde die Fettphase hinzugegeben und etwa 1 Minute lang homogenisiert. Nach dem Abkühlen auf etwa 30° C wurden Lipochroman-6 und die übrigen kosmetischen Wirkstoffe zugegeben und der pH-Wert mit Citronensäure auf einen Wert im Bereich von 4,8 - 5,2 eingestellt.

Anschließend wurden das Carbomer und das erfindungsgemäße nanoskalige Hydrogel hinzugegeben.

Die Applikation der Zusammensetzungen auf die Haut führte zu einer signifikanten Verringerung der Faltentiefe.

Eine Applikation der Zusammensetzungen auf raue Haut führte zu eine signifikanten Verbesserung des Hauterscheinungsbildes; die Haut fühlte sich geschmeidiger, weicher und glatter an.

Die Applikation der Zusammensetzungen auf trockene Haut führte zu einer signifikanten Verbesserung der Hautfeuchtigkeit, die auch noch mehrere Stunden nach der Applikation nachweisbar war.

**Liste der verwendeten Inhaltsstoffe**

| **Komponente** | **INCI-Bezeichung** | | **Hersteller** |
|---|---|---|---|
| Aristoflex^{®} AVC | Ammonium Acryloyldimethyltaurate/Vinylpyrrolid one Copolymer, t-Butyl Alcohol | | Clariant |
| Baysilonöl^{®} M 350 | Dimethicone | Polydimethylsiloxan | GE-Bayer-Silicones |
| Controx^{®} KS | Tocopherol, Hydrogenated Palm Glycerides Citrate | Tocopherol-Mono-diglyceride-citrat-Gemisch | Cognis |
| Cutina^{®} GMS-V | Glyceryl Stearate | Glycerin-mono-dipalmitat-stearat, pflanzlich | Cognis |
| Dow Corning^{®} 9040 | Cyclomethicone, Dimethicone Crosspolymer | | Dow Corning |
| Dow Coming^{®} 593 | Dimethicone Trimethylsiloxysilicate | | Dow Corning |
| Eusolex^{®} 4360 | Benzophenone-3 | | Merck |
| Eusolex^{®} 6300 | 4-Methylbenzylidene Camphor | | Merck |
| Fucogel 1000 | Biosaccharide Gum-1 | | Solabia |
| Granlux^{®} MSN 50 | Titanium dioxide, Apricot Kernel Oil PEG-40 Esters, Cetearyl Glucoside, Polydecene | Dispersion mit 45 - 55 Gew.-% TiO₂, Partikel-größe 22 nm | Oy Granula AB, Finnland |
| Hibiscin^{®} HP-LS-9198 | Water, Hibiscus esculentus Seed Extract, Phenoxyethanol | | Laboratoires Sérobiologiques |
| Kessco^{®} IPS | Isopropyl Stearate | | Akzo Nobel |
| Lanette^{®} 22 | Behenyl Alcohol | | Cognis |
| Lipochroman-6 | Dimethylmethoxy Chromanol | 4-Dihydro-7-methoxy-2,2-dimethyl-2H-1-benzopyran-6-ol | Lipotec S.A: |
| Myritol^{®} PC | Propylene Glycol Dicaprylate/Dicaprate | | Cognis |
| Parsol^{®} 1789 | Butyl Methoxydibenzoylmethane | | Roche-Givaudan |
| Parsol^{®} MCX | Octyl Methoxycinnamate | | Roche-Givaudan |
| Sepigel^{®} 305 | Polyacrylamide, C13-14 Isoparaffin, Laureth-7 | | SEPPIC |
| Simulgel^{®} NS | Hydroxyethyl Acrylate/Sodium Acryloyldimethyltaurate Copolymer, Squalane, Polysorbate-60 | | SEPPIC |
| Stenol^{®} 16/18 | Cetearyl Alcohol | Cetyl-/Stearylalkohol (1 : 2) | Cognis |
| Uvinul^{®} T 150 | Octyl Triazone | | BASF |
| V-Protein flüssig (COS 152/22-A) | Water, Propylene Glycol, Hydrolyzed Pea Protein (Pisum sativum) | | Cosmetochem |

## Patentansprüche

1. Verwendung von nanoskaligen Hydrogelen aus Polymeren mit einem mittleren Teilchendurchmesser von 10 - 400 nm und einer LCST (Lower Critical Solution Temperature, untere kritische Entmischungstemperatur) von 28 - 38 °C, ausgewählt aus Poly-N-isopropylacrylamiden, Polyvinylethern, Polyacrylaten, Polymethacrylaten, Polyacrylamiden, Polymethacrylamiden, Polyurethanen, Polyvinylpyrrolidonen und Polyvinylalkoholen sowie deren Copolymeren, alkylierten und/oder hydroxyalkylierten Polysacchariden und Celluloseethern, in einem zur topischen Anwendung geeigneten Träger mit einem Feststoffgehalt an LCST-Polymer von 0,5-1,5 Gew.-% zur nicht-therapeutischen Behandlung
- von Falten und Fältchen
- rauer Haut und/oder
- trockener Haut.

2. Kosmetische Zusammensetzung, enthaltend in einem zur topischen Anwendung geeigneten Träger
a) 0,5-1,5 Gew.-% mindestens eines nanoskaligen Hydrogels aus mindestens einem Polymer mit einem mittleren Teilchendurchmesser von 10 - 400 nm, das eine LCST (Lower Critical Solution Temperature, untere kritische Entmischungstemperatur) von 28 - 38 °C aufweist und ausgewählt ist aus Poly-N-isopropylacrylamiden, Polyvinylethern, Polyacrylaten, Polymethacrylaten, Polyacrylamiden, Polymethacrylamiden, Polyurethanen, Polyvinylpyrrolidonen und Polyvinylalkoholen sowie deren Copolymeren, alkylierten und/oder hydroxyalkylierten Polysacchariden und Celluloseethern,
sowie weiterhin
b) mindestens ein verdickendes Polymer, das ausgewählt ist aus teilweise oder vollständig neutralisierten, gegebenenfalls vernetzten Polymeren aus mindestens einem der Monomere Acrylsäure, Methacrylsäure, Itaconsäure, Maleinsäure und 2-Methyl-2[(1-oxo-2-propenyl)-amino]-1-propansulfonsäure, das mindestens ein neutrales Comonomer, ausgewählt aus Vinylpyrrolidon, Acrylamid und den C₁-C₃₀-Estern von Acrylsäure, Methacrylsäure, Itaconsäure und Maleinsäure, enthalten kann.

3. Kosmetische Zusammensetzung, enthaltend in einem zur topischen Anwendung geeigneten Träger
a) 0,5-1,5 Gew.-% mindestens eines nanoskaligen Hydrogels aus mindestens einem Polymer mit einem mittleren Teilchendurchmesser von 10 - 400 nm, das eine LCST (Lower Critical Solution Temperature, untere kritische Entmischungstemperatur) von 28 - 38 °C aufweist und ausgewählt ist aus Poly-N-isopropylacrylamiden, Polyvinylethern, Polyacrylaten, Polymethacrylaten, Polyacrylamiden, Polymethacrylamiden, Polyurethanen, Polyvinylpyrrolidonen und Polyvinylalkoholen sowie deren Copolymeren, alkylierten und/oder hydroxyalkylierten Polysacchariden und Celluloseethern,
sowie weiterhin
b) mindestens eine Siliconverbindung, die ausgewählt ist aus Decamethylcyclopentasiloxan, Dodecamethylcyclohexasiloxan und Siliconpolymeren, die gewünschtenfalls quervernetzt sein können.

4. Kosmetische Zusammensetzung, enthaltend in einem zur topischen Anwendung geeigneten Träger
a) 0,5-1,5 Gew.-% mindestens eines nanoskaligen Hydrogels aus mindestens einem Polymer mit einem mittleren Teilchendurchmesser von 10 - 400 nm, das eine LCST (Lower Critical Solution Temperature, untere kritische Entmischungstemperatur) von 28 - 38 °C aufweist und ausgewählt ist aus Poly-N-isopropylacrylamiden, Polyvinylethern, Polyacrylaten, Polymethacrylaten, Polyacrylamiden, Polymethacrylamiden, Polyurethanen, Polyvinylpyrrolidonen und Polyvinylalkoholen sowie deren Copolymeren, alkylierten und/oder hydroxyalkylierten Polysacchariden und Celluloseethern,
sowie weiterhin
b) mindestens ein Effektpigment oder Lichtschutzpigment.

5. Kosmetische Zusammensetzung, enthaltend in einem zur topischen Anwendung geeigneten Träger
a) 0,5-1,5 Gew.-% mindestens eines nanoskaligen Hydrogels aus mindestens einem Polymer mit einem mittleren Teilchendurchmesser von 10 - 400 nm, das eine LCST (Lower Critical Solution Temperature, untere kritische Entmischungstemperatur) von 28 - 38 °C aufweist und ausgewählt ist aus Poly-N-isopropylacrylamiden, Polyvinylethern, Polyacrylaten, Polymethacrylaten, Polyacrylamiden, Polymethacrylamiden, Polyurethanen, Polyvinylpyrrolidonen und Polyvinylalkoholen sowie deren Copolymeren, alkylierten und/oder hydroxyalkylierten Polysacchariden und Celluloseethern,
sowie weiterhin
b) mindestens ein Proteinhydrolysat.

6. Kosmetische Zusammensetzung, enthaltend in einem zur topischen Anwendung geeigneten Träger
a) 0,5-1,5 Gew.-% mindestens eines nanoskaligen Hydrogels aus mindestens einem Polymer mit einem mittleren Teilchendurchmesser von 10 - 400 nm, das eine LCST (Lower Critical Solution Temperature, untere kritische Entmischungstemperatur) von 28 - 38 °C aufweist und ausgewählt ist aus Poly-N-isopropylacrylamiden, Polyvinylethern, Polyacrylaten, Polymethacrylaten, Polyacrylamiden, Polymethacrylamiden, Polyurethanen, Polyvinylpyrrolidonen und Polyvinylalkoholen sowie deren Copolymeren, alkylierten und/oder hydroxyalkylierten Polysacchariden und Celluloseethern,
sowie weiterhin
b) mindestens ein Mono-, Oligo- oder Polysaccharid.

7. Kosmetische Zusammensetzung, enthaltend in einem zur topischen Anwendung geeigneten Träger
a) 0,5-1,5 Gew.-% mindestens eines nanoskaligen Hydrogels aus mindestens einem Polymer mit einem mittleren Teilchendurchmesser von 10 - 400 nm, das eine LCST (Lower Critical Solution Temperature, untere kritische Entmischungstemperatur) von 28 - 38 °C aufweist und ausgewählt ist aus Poly-N-isopropylacrylamiden, Polyvinylethern, Polyacrylaten, Polymethacrylaten, Polyacrylamiden, Polymethacrylamiden, Polyurethanen, Polyvinylpyrrolidonen und Polyvinylalkoholen sowie deren Copolymeren, alkylierten und/oder hydroxyalkylierten Polysacchariden und Celluloseethern,
sowie weiterhin
b) mindestens eine α-Hydroxycarbonsäure oder α-Ketocarbonsäure oder deren Ester-, Lacton- oder Salzform.

## Claims

1. Use of nanoscale hydrogels of polymers with an average particle diameter of 10-400 nm and an LCST (lower critical solution temperature) of 28-38°C, selected from poly-N-isopropylacrylamides, polyvinyl ethers, polyacrylates, polymethacrylates, polyacrylamides, polymethacrylamides, polyurethanes, polyvinylpyrrolidones and polyvinyl alcohols, and copolymers thereof, alkylated and/or hydroxyalkylated polysaccharides and cellulose ethers, in a carrier suitable for topical application having a solids content of LCST polymer of 0.5-1.5% by weight for the nontherapeutic treatment
- of wrinkles and lines
- of rough skin and/or
- of dry skin.

2. Cosmetic composition comprising, in a carrier suitable for topical application,
a) 0.5-1.5% by weight of at least one nanoscale hydrogel of at least one polymer with an average particle diameter of 10-400 nm, which has an LCST (lower critical solution temperature) of 28-38°C and is selected from poly-N-isopropylacrylamides, polyvinyl ethers, polyacrylates, polymethacrylates, polyacrylamides, polymethacrylamides, polyurethanes, polyvinylpyrrolidones and polyvinyl alcohols, and copolymers thereof, alkylated and/or hydroxyalkylated polysaccharides and cellulose ethers,
and also
b) at least one thickening polymer which is selected from partially or completely neutralized, optionally crosslinked polymers of at least one of the monomers acrylic acid, methacrylic acid, itaconic acid, maleic acid and 2-methyl-2-[(1-oxo-2-propenyl)amino]-1-propanesulphonic acid, which can comprise at least one neutral comonomer selected from vinylpyrrolidone, acrylamide and the C₁-C₃₀-esters of acrylic acid, methacrylic acid, itaconic acid and maleic acid.

3. Cosmetic composition comprising, in a carrier suitable for topical application,
a) 0.5-1.5% by weight of at least one nanoscale hydrogel of at least one polymer with an average particle diameter of 10-400 nm, which has an LCST (lower critical solution temperature) of 28-38°C and is selected from poly-N-isopropylacrylamides, polyvinyl ethers, polyacrylates, polymethacrylates, polyacrylamides, polymethacrylamides, polyurethanes, polyvinylpyrrolidones and polyvinyl alcohols, and copolymers thereof, alkylated and/or hydroxyalkylated polysaccharides and cellulose ethers,
and also
b) at least one silicone compound which is selected from decamethylcyclopentasiloxane, dodecamethylcyclohexasiloxane and silicone polymers, which may if desired be crosslinked.

4. Cosmetic composition comprising, in a carrier suitable for topical application,
a) 0.5-1.5% by weight of at least one nanoscale hydrogel of at least one polymer with an average particle diameter of 10-400 nm which has an LCST (lower critical solution temperature) of 28-38°C and is selected from poly-N-isopropylacrylamides, polyvinyl ethers, polyacrylates, polymethacrylates, polyacrylamides, polymethacrylamides, polyurethanes, polyvinylpyrrolidones and polyvinyl alcohols, and copolymers thereof, alkylated and/or hydroxyalkylated polysaccharides and cellulose ethers,
and also
b) at least one effect pigment or photoprotective pigment.

5. Cosmetic composition comprising, in a carrier suitable for topical application,
a) 0.5-1.5% by weight of at least one nanoscale hydrogel of at least one polymer with an average particle diameter of 10-400 nm which has an LCST (lower critical solution temperature) of 28-38°C and is selected from poly-N-isopropylacrylamides, polyvinyl ethers, polyacrylates, polymethacrylates, polyacrylamides, polymethacrylamides, polyurethanes, polyvinylpyrrolidones and polyvinyl alcohols, and copolymers thereof, alkylated and/or hydroxyalkylated polysaccharides and cellulose ethers,
and also
b) at least one protein hydrolysate.

6. Cosmetic composition comprising, in a carrier suitable for topical application,
a) 0.5-1.5% by weight of at least one nanoscale hydrogel of at least one polymer with an average particle diameter of 10-400 nm which has an LCST (lower critical solution temperature) of 28-38°C and is selected from poly-N-isopropylacrylamides, polyvinyl ethers, polyacrylates, polymethacrylates, polyacrylamides, polymethacrylamides, polyurethanes, polyvinylpyrrolidones and polyvinyl alcohols, and copolymers thereof, alkylated and/or hydroxyalkylated polysaccharides and cellulose ethers,
and also
b) at least one mono-, oligo- or polysaccharide.

7. Cosmetic composition comprising, in a carrier suitable for topical application,
a) 0.5-1.5% by weight of at least one nanoscale hydrogel of at least one polymer with an average particle diameter of 10-400 nm which has an LCST (lower critical solution temperature) of 28-38°C and is selected from poly-N-isopropylacrylamides, polyvinyl ethers, polyacrylates, polymethacrylates, polyacrylamides, polymethacrylamides, polyurethanes, polyvinylpyrrolidones and polyvinyl alcohols, and copolymers thereof, alkylated and/or hydroxyalkylated polysaccharides and cellulose ethers,
and also
b) at least one α-hydroxycarboxylic acid or α-ketocarboxylic acid or ester, lactone or salt form thereof.

## Revendications

1. Utilisation d'hydrogels à l'échelle du nanomètre, constitués de polymères possédant un diamètre moyen de particule de 10 à 400 nm et une valeur LCST (Lower Critical Solution Température, température critique de dissolution inférieure) de 28 à 38 °C, choisis parmi des poly-N-isopropylacrylamides, des éthers polyvinyliques, des polyacrylates, des polyméthacrylates, des polyacrylamides, des polyméthacrylamides, des polyuréthanes, des polyvinylpyrrolidones et des alcools polyvinyliques, ainsi que leurs copolymères, des polysaccharides alkylés et/ou hydroxyalkylés et des éthers cellulosiques, dans un support approprié pour l'application locale, avec une teneur en substances solides de polymères LCST de 0,5 à 1,5 % en poids, à des fins de traitement non thérapeutique
- de rides et de ridules ;
- d'une peau rugueuse et/ou
- d'une peau sèche.

2. Composition cosmétique contenant, dans un support approprié pour l'application locale :
a) à concurrence de 0,5 à 1,5 % en poids, au moins un hydrogel à l'échelle du nanomètre d'au moins un polymère comprenant un diamètre moyen de particule de 10 à 400 nm, qui présente une valeur LCST (Lower Critical Solution Temperature, température critique de dissolution inférieure) de 28 à 38 °C et qui est choisi parmi des poly-N-isopropylacrylamides, des éthers polyvinyliques, des polyacrylates, des polyméthacrylates, des polyacrylamides, des polyméthacrylamides, des polyuréthanes, des polyvinylpyrrolidones et des alcools polyvinyliques, ainsi que leurs copolymères, des polysaccharides alkylés et/ou hydroxyalkylés et des éthers cellulosiques,
et en outre
b) au moins un polymère épaississant qui est choisi parmi des polymères partiellement ou complètement neutralisés, éventuellement réticulés, d'au moins un des monomères que sont l'acide acrylique, l'acide méthacrylique, l'acide itaconique, l'acide maléique et l'acide 2-méthyl-2-[(1-oxo-2-propényl)-amino]-1-propanesulfonique, qui peut contenir au moins un comonomère neutre choisi parmi la vinylpyrrolidone, l'acrylamide et les esters en C₁-C₃₀ de l'acide acrylique, de l'acide méthacrylique, de l'acide itaconique et de l'acide maléique.

3. Composition cosmétique, contenant, dans un support approprié pour l'application locale :
a) à concurrence de 0,5 à 1,5 % en poids, au moins un hydrogel à l'échelle du nanomètre d'au moins un polymère comprenant un diamètre moyen de particule de 10 à 400 nm, qui présente une valeur LCST (Lower Critical Solution Température, température critique de dissolution inférieure) de 28 à 38 °C et qui est choisi parmi des poly-N-isopropylacrylamides, des éthers polyvinyliques, des polyacrylates, des polyméthacrylates, des polyacrylamides, des polyméthacrylamides, des polyuréthanes, des polyvinylpyrrolidones et des alcools polyvinyliques, ainsi que leurs copolymères, des polysaccharides alkylés et/ou hydroxyalkylés et des éthers cellulosiques,
et en outre
b) au moins un composé de silicone qui est choisi parmi le décaméthylcyclopentasiloxane, le dodécaméthylcyclohexasiloxane et des polymères de silicone qui peuvent être réticulés si on le souhaite.

4. Composition cosmétique, contenant, dans un support approprié pour l'application locale :
a) à concurrence de 0,5 à 1,5 % en poids, au moins un hydrogel à l'échelle du nanomètre d'au moins un polymère comprenant un diamètre moyen de particule de 10 à 400 nm, qui présente une valeur LCST (Lower Critical Solution Température, température critique de dissolution inférieure) de 28 à 38 °C et qui est choisi parmi des poly-N-isopropylacrylamides, des éthers polyvinyliques, des polyacrylates, des polyméthacrylates, des polyacrylamides, des polyméthacrylamides, des polyuréthanes, des polyvinylpyrrolidones et des alcools polyvinyliques, ainsi que leurs copolymères, des polysaccharides alkylés et/ou hydroxyalkylés et des éthers cellulosiques,
et en outre
b) au moins un pigment d'effet ou un pigment de protection contre l'effet de la lumière.

5. Composition cosmétique, contenant, dans un support approprié pour l'application locale :
a) à concurrence de 0,5 à 1,5 % en poids, au moins un hydrogel à l'échelle du nanomètre d'au moins un polymère comprenant un diamètre moyen de particule de 10 à 400 nm, qui présente une valeur LCST (Lower Critical Solution Température, température critique de dissolution inférieure) de 28 à 38 °C et qui est choisi parmi des poly-N-isopropylacrylamides, des éthers polyvinyliques, des polyacrylates, des polyméthacrylates, des polyacrylamides, des polyméthacrylamides, des polyuréthanes, des polyvinylpyrrolidones et des alcools polyvinyliques, ainsi que leurs copolymères, des polysaccharides alkylés et/ou hydroxyalkylés et des éthers cellulosiques,
et en outre
b) au moins un hydrolysat protéique.

6. Composition cosmétique, contenant, dans un support approprié pour l'application locale :
a) à concurrence de 0,5 à 1,5 % en poids, au moins un hydrogel à l'échelle du nanomètre d'au moins un polymère comprenant un diamètre moyen de particule de 10 à 400 nm, qui présente une valeur LCST (Lower Critical Solution Température, température critique de dissolution inférieure) de 28 à 38 °C et qui est choisi parmi des poly-N-isopropylacrylamides, des éthers polyvinyliques, des polyacrylates, des polyméthacrylates, des polyacrylamides, des polyméthacrylamides, des polyuréthanes, des polyvinylpyrrolidones et des alcools polyvinyliques, ainsi que leurs copolymères, des polysaccharides alkylés et/ou hydroxyalkylés et des éthers cellulosiques,
et en outre
b) au moins un monosaccharide, un oligosaccharide ou un polysaccharide.

7. Composition cosmétique, contenant, dans un support approprié pour l'application locale :
a) à concurrence de 0,5 à 1,5 % en poids, au moins un hydrogel à l'échelle du nanomètre d'au moins un polymère comprenant un diamètre moyen de particule de 10 à 400 nm, qui présente une valeur LCST (Lower Critical Solution Température, température critique de dissolution inférieure) de 28 à 38 °C et qui est choisi parmi des poly-N-isopropylacrylamides, des éthers polyvinyliques, des polyacrylates, des polyméthacrylates, des polyacrylamides, des polyméthacrylamides, des polyuréthanes, des polyvinylpyrrolidones et des alcools polyvinyliques, ainsi que leurs copolymères, des polysaccharides alkylés et/ou hydroxyalkylés et des éthers cellulosiques,
et en outre
b) au moins un acide α-hydroxycarboxylique ou un acide α-cétocarboxylique ou leur forme d'ester, de lactone ou de sel.
